# EUROPEAN PATENT SPECIFICATION

(11) **EP 3 616 558 B1**
(45) Date of publication and mention of the grant of the patent: **25.08.2021**
(21) Application number: 18796080.2
(22) Date of filing: 24.04.2018
(51) Int. Cl.: A45F 3/02

(54) **BACKPACK**
RUCKSACK
SAC À DOS

(30) Priority: 25.04.2017 ES 201700386 U
(43) Date of publication of application: 04.03.2020
(73) Proprietor: WOMAN'SBACK, S.L., 08027 Barcelona (ES)
(72) Inventor: PRANDI CHEVALIER, Maria, 08290 Cerdanyola del Valles (Barcelona) (ES)
(74) Representative: Curell Suñol S.L.P.
(86) International application number: PCT/ES2018/000047
(87) International publication number: WO 2018/211157

(56) References cited:
- FR-A1- 2 849 999
- JP-A- H09 173 120
- JP-A- 2005 312 840
- US-A- 4 887 751
- US-A1- 2002 153 401
- US-A1- 2013 277 405

## Description

### Object of the invention.

The object of this invention is a backpack comprising a body with at least one compartment for carrying various objects and straps for fastening the backpack to the user's body; the backpack has features designed for carrying in a conventional position, that is, on the back, without exerting any pressure on the shoulder and chest area on one side of the user's body.

### Field of application of the invention.

This invention can be used during any type of exercise, particularly by women who have had a partial or total mastectomy and/or a lymph node dissection in the chest and underarm area and, in general, by any individual suffering from a disease that advises against applying pressure on the shoulder or chest area on one side of the body.

### Prior art.

Diagnosed cases of breast cancer indicate that this type of cancer will affect one in eight women at some time in their lives. Often, the medical diagnosis indicates a partial or total mastectomy and a lymph node dissection through which some or all of the lymph nodes are removed from the chest and underarm area.

This removal involves the possibility of developing lymphedema due to an acquired interruption or damage to the axillary lymphatic system. The volume of lymph that is produced exceeds the system's drainage capacity, causing an accumulation of fluid and arm pain. Protecting the shoulder area, armpit, pectoral and dorsal areas near the chest from any pressure or strain plays a major role in preventing lymphedema, which is why doctors expressly prohibit patients from carrying backpacks that can apply pressure from shoulder straps.

On the other hand, exercise reduces the risk of relapse and CRF (Cancer Related Fatigue) associated with the disease and its treatment by 50%. Exercise is, therefore, a useful course of action for breast cancer patients, and participating in mountain sports is a recommended activity.

Existing backpacks are currently designed with at least one compartment for carrying various objects and two shoulder straps for support, which is unadvisable with certain pathologies like those indicated above.

Also popular are backpacks that use a single cross-body strap that crosses over the chest; however, these backpacks have a very limited capacity and do not provide stable support for the body, nor the safety features necessary for practicing mountain sports.

Documents JP 2005 312840 A, JP H09 173120 A, US 4 887 751 A, US 2013/277405 A1 and FR 2 849 999 A1 disclose examples of backpacks having only one strap shoulder.

The applicant of this invention is unaware of the existence of backpacks designed to meet this need at a time when there is a growing number of women of active age interested in exercise, specifically backpacks designed to protect women from the risk of lymphedema in the arm after having a lymph node dissection.

Therefore, the technical issue raised is the development of a backpack that protects women who have had lymph nodes removed from the chest and underarm area from the risk of lymphedema in the arm while allowing them to resume mountain sports, and which is suitable for those individuals who have been advised against exerting pressure on an adjacent shoulder or chest or dorsal area.

### Invention specifications.

The backpack of this invention has a main body comprising at least one compartment for carrying various objects and straps to fasten the backpack to the user's body and has constructive features designed to protect the front and back of the chest as well as the shoulder from pressure and strain, fully limiting the elements that can lead to the generation of lymphedema in the arm in the cases mentioned above.

The backpack of the invention has the particularity of having a main support strap on one of the shoulders, the user's right or left, and does not have a second support strap on the other left or right shoulder, in order to keep pressure off the shoulder and the adjacent chest area.

This backpack also comprises a pectoral stabilizer that is located below the chest of the affected side, instead of at the same height, and which is attached at opposite ends to an upper section of the main shoulder strap and to a lower section on the opposite side of the backpack. This front strap ensures backpack stability and fit without any pressure or weight on the entire shoulder area, left or right, on the affected side.

Another feature of the backpack of the invention is that it has a reinforced hip belt designed to support the backpack's weight. The backpack's hip support is specifically reinforced, since this is where most of the weight of the backpack will fall.

Another feature of the backpack of the invention is that it further comprises a second side fastening strap, which is attached at end to one side of the backpack, opposite the side with the main support strap over the user's shoulder, and on the other side to a section of the hip belt located on the same side as the main shoulder strap; therefore, when in use the second strap crosses the body laterally, below the user's armpit and chest area, without putting pressure on that same chest area or the shoulder on the same side.

Therefore, to summarize, the features of the backpack that make it possible to solve the issue are the features defined in appended claim 1.

In one embodiment, the backpack has symmetrical hooks on both sides that make it possible to alternately attach the main shoulder support strap to the corresponding front strap of the pectoral stabilizer, in two symmetrical positions on either side, depending on which side the user wants to relieve the pressure of a shoulder strap off the shoulder and chest area.

### Description of the figures.

To supplement the description and in order to facilitate the understanding of the characteristics of the invention, a set of illustrative figures, including but not limited to the following, is attached to this specification:
- Figure 1 is a vertical front view of an example of the backpack not falling under the scope of invention as defined by the appended claims
- Figure 2 shows a schematic view of the backpack in the previous figure in a position of use.
- Figure 3 shows a schematic front vertical view of an embodiment of the backpack of the invention, including a second fastening strap.
- Figure 4 shows a schematic view of the backpack in Figure 3 when in use, in which the placement of the second strap can be observed below the armpit and the chest of the user.

### Descriptions of the preferred embodiments.

In the example shown of the backpack, referenced as a whole as one that (1) comprises a main strap (2) so it can be supported by one of the user's shoulders, in this particular example the left shoulder; the backpack does not have a second support strap on the other shoulder (the right), keeping all pressure off the second shoulder (H).

Obviously, depending on the needs of the user, the first strap (2) could be conveniently positioned on the backpack to rest on the right shoulder, releasing pressure from the left shoulder (H).

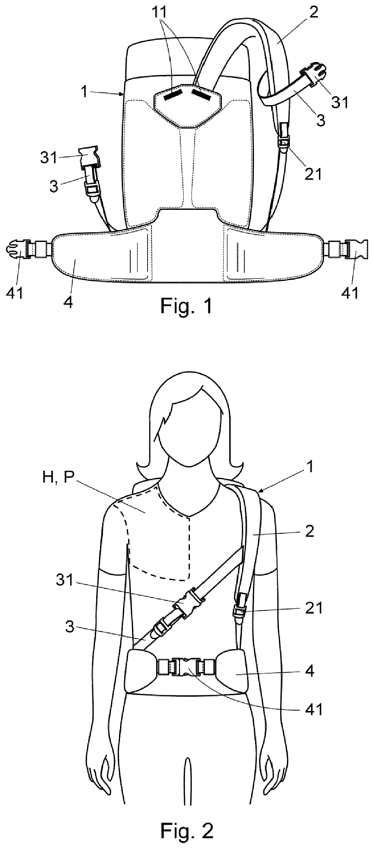

The backpack (1) further comprises a pectoral stabilizer comprising a front strap (3) attached at opposite ends to an upper section of the main support strap (2) on the left shoulder and to a lower section on the right, opposite side of the backpack (1), so that in the position of use shown in Figure 2, the front strap (3) of the pectoral stabilizer is attached across the body like a shoulder strap, under the user's chest, keeping pressure from being place on the user's right chest area (P).

This pectoral stabilizer helps to keep the backpack tightly fitted to the user's back.

Since it has a single main support strap (2) on one of the shoulders, the backpack (1) also has a hip belt (4) that has been specially reinforced to attach the backpack to the hip and to support most of the weight of the backpack, since it is precisely in this area where most of the weight will fall.

Both the main support strap (2) on one of the shoulders and the front strap (3) of the pectoral stabilizer will have the conventional systems to regulate strap length and adjustment to the user's individual physical characteristics.

In the example shown in fig.1, the backpack (1) has symmetrical hooks (11) on both sides that make it possible to alternately attach the main strap (2) to the corresponding front strap (3) of the pectoral stabilizer, in two symmetrical positions on either side, depending on the which side the user wants to relieve pressure (2, 3) off the shoulder (H) and chest area (P).

As shown in the attached figures, the first strap (2) is placed on one side of the backpack and both the main shoulder strap (2) and the front strap (3) and hip belt (4) comprise closures (21, 31, 41) that can regulate the length.

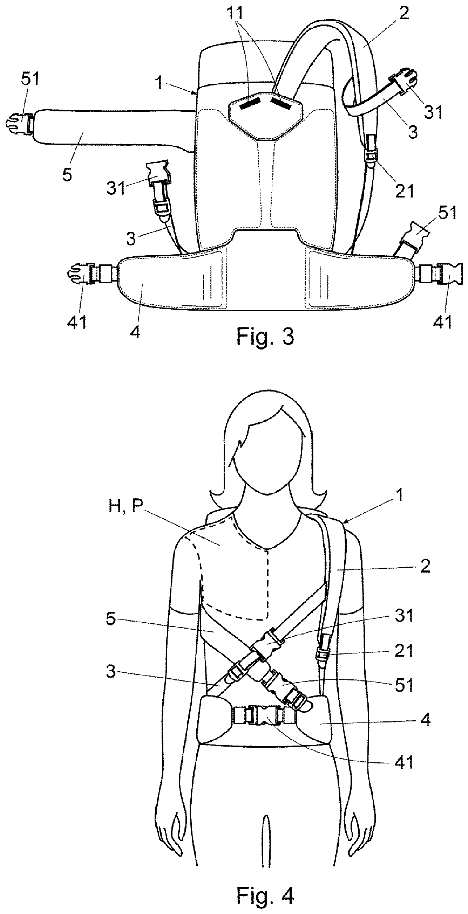

In the embodiment of the present invention shown in Figures 3 and 4, the backpack additionally comprises a second strap (5) to laterally attach the backpack (1), mainly from the side of the backpack opposite to the attachment for the main strap (2).

This second strap (5) is attached at one end to one side of the backpack (1) on the opposite side of the side that has the main shoulder strap (2), and on the other end to a section of the hip belt (4) located on the same side as the main shoulder strap (2), so that the position of this second strap (5) when in use crosses laterally, below the user's armpit and chest area (P), without putting pressure on that same chest area (P) or the shoulder (H) on the same side.

This second strap (5) also has a middle closure (51) with length adjustment.

Having thoroughly described the nature of the invention, as well as a preferred embodiment, it is hereby stated for pertinent purposes that the materials, shape, size and arrangement of the described elements may be modified, provided that such modifications fall under the scope of protection defined by the appended claims.

## Claims

1. Backpack; comprising a body with at least one compartment for carrying various objects, and straps so that the user can carry the backpack; **wherein the backpack comprises:**
- a main support strap (2) on one of the shoulders and no second shoulder strap;
- a pectoral stabilizer comprised by a front strap (3) which is attached at opposite ends to an upper section of the main shoulder strap (2) on one of the shoulders, and to a lower section on the opposite side of the backpack (1), so that when in use it leaves the shoulder (H) and chest area (P) on one side of the user's body free of pressure; and
- a specially reinforced hip belt (4), suitable for attaching the backpack to the hip and supporting most of the weight of the backpack;
**characterized in that** the backpack further comprises a second strap (5) laterally attached to the backpack (1), which is attached at one end to one side of the backpack (1) on the opposite side of the side that has the main shoulder strap (2), and on the other end to a section of the hip belt (4) located on the same side as the main shoulder strap (2), so that the position of this second strap (5) when in use crosses laterally, below the user's armpit and chest area (P), without putting pressure on that same chest area (P) or the shoulder (H) on the same side.

2. Backpack, according to claim 1, **characterized in that** the second strap (5) has a middle closure (51) with length adjustment.

3. Backpack, according to Claim 1 or 2, **characterized in that** the backpack (1) has symmetrical hooks (11) on either side that make it possible to alternately attach the main shoulder support strap (2) to the corresponding front strap (3) of the chest stabilizer, in two symmetrical positions on either side;
wherein the main strap is attached to one side of the backpack;
wherein the main shoulder strap (2), the front strap (3) and the hip belt (4) comprise closures (21, 31, 41) with length adjustment.

## Patentansprüche

1. Rucksack; umfassend einen Körper mit mindestens einem Fach zum Tragen verschiedener Gegenstände, und Gurte, so dass der Benutzer den Rucksack tragen kann; wobei der Rucksack umfasst:
- ein Hauptstützband (2) an einer der Schultern und keinen zweiten Schultergurt;
- einen Bruststabilisator, der ein vorderes Band (3) umfasst, der an gegenüberliegenden Enden an einem oberen Abschnitt des Hauptschulterbandes (2) auf einer der Schultern, und an einem unteren Abschnitt auf der gegenüberliegenden Seite des Rucksacks (1) befestigt ist, so dass er im Gebrauch den Schulter (H) und Brustbereich (P) auf einer Seite des Körpers des Benutzers frei von Druck lässt; und
- einen speziell verstärkten Hüftgurt (4), der geeignet ist, den Rucksack an der Hüfte zu befestigen und den größten Teil des Gewichts des Rucksacks zu tragen;
**dadurch gekennzeichnet, dass** der Rucksack ferner ein zweites Band (5) umfasst, der seitlich am Rucksack (1) befestigt ist und an einem Ende an einer Seite des Rucksacks (1) auf der Seite befestigt ist, die der Seite gegenüberliegt, die das Hauptschulterband (2) aufweist, und am anderen Ende an einem Abschnitt des Hüftgurtes (4), der sich auf derselben Seite wie das Hauptschulterband (2) befindet, befestigt ist, so dass die Position dieses zweiten Band (5) im Gebrauch seitlich unter der Achselhöhle und dem Brustbereich (P) des Benutzers quert, ohne Druck auf denselben Brustbereich (P) oder die Schulter (H) auf derselben Seite auszuüben.

2. Rucksack nach Anspruch 1, **dadurch gekennzeichnet, dass** das zweite Band (5) einen Mittelverschluss (51) mit Längenverstellung aufweist.

3. Rucksack nach Anspruch 1 oder 2, **dadurch gekennzeichnet, dass** der Rucksack (1) auf beiden Seiten symmetrische Aufhänger (11) aufweist, die es ermöglichen, das Hauptschulterstützband (2) abwechselnd an dem entsprechenden vorderen Band (3) des Bruststabilisators zu befestigen, und zwar in zwei symmetrischen Positionen auf jeder Seite;
wobei der Hauptgurt an einer Seite des Rucksacks befestigt ist;
wobei das Hauptschulterband (2), das vordere Band (3) und der Hüftgurt (4) Verschlüsse (21, 31, 41) mit Längenverstellung aufweisen.

## Revendications

1. Sac-à-dos; comprenant un corps avec au moins un compartiment pour porter divers objets ainsi que des bretelles afin que l'usager puisse porter le sac-à-dos; ledit sac-à-dos comprenant:
- une bretelle principale (2) de support sur l'une des épaules et pas bretelle de deuxième épaule;
- un stabilisateur pectoral composé d'une bretelle frontale qui est fixée par des extrémités opposées à une zone supérieure de la bretelle principale (2) d'épaule sur l'une des épaules, et à une zone inférieure sur le côté opposé du sac-à-dos (1), de manière que pendant l'utilisation il laisse libres de pression l'épaule (H) et la zone de la poitrine (P) sur un côté du corps de l'usager; et
- une ceinture de hanche (4) spécialement renforcée, adaptée pour fixer le sac-à-dos à la hanche et supporter la plupart du poids du sac-à-dos;
**caractérisé en ce que** le sac-à-dos comprend en outre une deuxième bretelle (5) fixée latéralement au sac-à-dos (1), qui est fixée par un extrémité à un côté du sac-à-dos (1), du côté opposé au côté qui a la bretelle principale (2) d'épaule, et par l'autre extrémité à une zone de la ceinture de hanche (4) située sur le même côté que la bretelle principale (2) d'épaule, de façon que la position de cette deuxième bretelle (5), pendant l'utilisation, croise latéralement, sous la zone de l'aisselle et de la poitrine (P) de l'usager, sans appliquer une pression sur cette même poitrine (P) ou l'épaule (H) du même côté.

2. Sac-à-dos selon la revendication 1, **caractérisé en ce que** la deuxième bretelle (5) a une fermeture intermédiaire (51) avec réglage de la longueur.

3. Sac-à-dos selon la revendication 1 ou 2, **caractérisé en ce que** le sac-à-dos (1) a des crochets symétriques (11) de chaque côté qui font qu'il soit possible de fixer alternativement la bretelle principale (2) de support d'épaule à la bretelle frontale (3) correspondante du stabilisateur de poitrine, dans deux positions symétriques de chaque côté;
dans lequel la bretelle principale est fixée à un côté du sac-à-dos;
dans lequel la bretelle principale (2) d'épaule, la bretelle frontale (3) et la ceinture de hanche (4) comprennent des fermetures (21, 31, 41) avec réglage de longueur.
